# EUROPEAN PATENT APPLICATION

(11) **EP 4 786 931 A2**
(43) Date of publication of application: **05.08.2026**
(21) Application number: 26150508.5
(22) Date of filing: 07.01.2026
(51) Int. Cl.: G01G 19/44, A61B 5/00

(54) **MEASURING WEIGHT IN A NEONATAL CARE SYSTEM WITH A SENSING ARRAY**

(30) Priority: 31.01.2025 US 202519042113
(71) Applicant: GE Precision Healthcare LLC, Waukesha, WI 53188 (US)
(72) Inventor: KAVOORI SETHUMADHAVAN, Nagapriya, Waukesha, 53188 (US); QUADROS, Vernon, Waukesha, 53188 (US); NAIK, Rajendra, Waukesha, 53188 (US)
(74) Representative: Kilburn & Strode LLP

(57) **Abstract**

A neonatal care system includes a processor and a memory. The memory includes instructions configured to cause the processor to receive strain amounts corresponding the strain sensors of a tactile sensor array. Additionally, the instructions cause the processor to generate a strain map comprising a visualization of the strain amounts in association with the corresponding X, Y coordinate locations of the strain sensors. Further, the instructions cause the processor to generate a weight map based on the strain map and a strain-weight model. Additionally, the weight map includes weights corresponding to the strain amounts. Further, the instructions cause the processor to identify a patient location. Additionally, the instructions cause the processor to determine a weight of the patient based on the patient location and the weight map

## Description

### BACKGROUND

The present disclosure generally relates to neonatal care systems and methods, and more particularly to systems and methods for a neonatal care system with integrated weighing.

Neonates, particularly premature infants, are often placed within neonatal care systems, such as an incubator and/or warmer, so that they may have a controlled and monitored environment to aid in their survival and growth. Accordingly, it is useful to monitor the infant's weight while the infant is in the incubator. It is additionally useful to monitor the infant's weight because medical therapies, such as the dosing of pharmeceuticals, are based upon an accurate determination of the infant's weight. Accordingly, neonatal care systems such as incubators and warmers may include integrated weighing systems to determine a weight of an infant in the neonatal care system.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

A neonatal care system includes a processor and a memory. The memory includes instructions configured to cause the processor to receive strain amounts corresponding the strain sensors of a tactile sensor array that is disposed beneath a patient that is disposed on a mattress. Additionally, the instructions cause the processor to generate a strain map comprising a visualization of the strain amounts in association with the corresponding X, Y coordinate locations of the strain sensors. Further, the instructions cause the processor to generate a weight map based on the strain map and a strain-weight model. Additionally, the weight map includes weights corresponding to the strain amounts. Further, the instructions cause the processor to identify a patient location. Additionally, the instructions cause the processor to determine a weight of the patient based on the patient location and the weight map.

In one embodiment, the instructions are executable by the processor to determine that the weight map indicates a presence of an additional uniform weight on the strain sensor array, determine an amount of the additional uniform weight, and subtract the uniform weight from each of the weights.

In one embodiment, the instructions are executable by the processor to determine that the strain map indicates a presence of an additional gradient strain on the strain sensor array, determine gradient strain increases associated with a sloping subset of the strains, subtract the gradient strain increases from the sloping subset of strains, and generate the weight map based on a strain map resulting from subtracting the gradient strain increases.

In one embodiment, the strain sensors include piezoelectric tactile sensors.

In one embodiment, identifying the patient location includes identifying a contiguous set of strain values that correspond to a predetermined threshold that is indicative of a strain that the patient places on corresponding strain sensors.

In one embodiment, determining that the predetermined threshold is indicative of the strain includes determining a density of the patient, determining a weight value threshold for a sensor based on the density and a number of sensors on which the patient is located, and determining that each of weights corresponding to the contiguous set of strain values is less than or equal to the weight value threshold.

In one embodiment, determining the weight of the patient includes summing a subset of weights of the weight map that are associated with a subset of strain sensors corresponding to the patient location.

In one embodiment, identifying the patient location includes capturing an image of the patient on the mattress, identifying the patient within the image, overlaying the image on the strain map, and identifying a subset of strain sensors corresponding to where the patient and the subset of strain sensors overlap.

A method includes receiving weights corresponding to load cells of a load cell array that is disposed beneath a patient that is disposed on a mattress. Additionally, the method includes identifying a patient location. Further, the method includes generating a weight map having the weights. Additionally, the method includes determining a weight of the patient based on the patient location and the weight map.

In one embodiment, the method includes determining that the weight map indicates a presence of an additional uniform weight on the load cell array. Additionally, the method includes determining an amount of the additional uniform weight. Further, the method includes subtracting the uniform weight from each of the weights.

In one embodiment, identifying the patient location includes capturing an image of the patient on a mattress. Further, identifying the patient location includes identifying an image location of the patient within the image. Additionally, identifying the patient location includes overlaying the image on the weight map. Further, identifying the patient location includes identifying a subset of the load cells that overlap with the image location of the patient in the overlaid image.

In one embodiment, identifying the patient location includes receiving strain amounts corresponding to strain sensors in a strain sensor array. Additionally, identifying the patient location includes generating a strain map comprising strain amounts associated with corresponding X, Y coordinate locations for the strain sensors. Further, identifying the patient location includes identifying a patient location based on the strain map, and identifying a subset of the load cells that correspond to a subset of the strain sensors that overlap with the patient location.

In one embodiment, identifying the patient location comprises identifying a contiguous set of strain values in the strain map that correspond to a predetermined threshold that is indicative of a strain that the patient places on a corresponding plurality of strain sensors.

In one embodiment, determining the weight of the patient includes summing a subset of the weights associated with a subset of strain sensors corresponding to the patient location.

A computer-readable storage medium includes instructions executable by a processor to receive strain amounts corresponding to strain sensors in a strain sensor array that is disposed beneath a patient that is disposed on a mattress. Additionally, the instructions are executable by the processor to generate a strain map having strain amounts associated with corresponding X, Y coordinate locations for the strain sensors. Further, the instructions are executable by the processor to generate a weight map based on the strain map and a strain-weight model, the weight map having weights corresponding to the strain amounts. Additionally, the instructions are executable by the processor to identify a patient location. Further, the instructions are executable by the processor to determine a weight of the patient based on the patient location and the weight map.

In one embodiment, the instructions are executable by the processor to determine that the weight map indicates a presence of an additional uniform weight on the strain sensor array, determine an amount of the additional uniform weight, and subtract the uniform weight from each of the weights.

In one embodiment, the instructions are executable by the processor to determine that the strain map indicates a presence of an additional gradient strain on the strain sensor array, determine gradient strain increases associated with a sloping subset of the strains, subtract the gradient strain increases from the sloping subset of strains, and generate the weight map based on a strain map resulting from subtracting the gradient strain increases.

In one embodiment, the strain sensors include piezoelectric tactile sensors.

In one embodiment, identifying the patient location includes identifying a contiguous set of strain values that correspond to a predetermined threshold that is indicative of a strain that the patient places on corresponding strain sensors.

Various other features, objects, and advantages of the invention will be made apparent from the following description taken together with the drawings.

### BRIEF DESCRIPTION OF THE DRAWINGS

The present disclosure is described with reference to the following Figures.
Fig. 1 is a perspective view of an exemplary neonatal care system that measures weight with a sensing array according to one embodiment of the present disclosure.
Fig. 2 is a perspective view of an exemplary neonatal care system that measures weight with a sensing array according to one embodiment of the present disclosure.
Fig. 3A is a side view of an example sensing array, patient, extraneous object, and mattress, according to one embodiment of the present disclosure.
Fig. 3B is a top view of the example sensing array, patient, extraneous objects, and mattress, according to one embodiment of the present disclosure.
Fig. 4A is an example strain map of the amount of force applied to each sensor in the sensing array, according to one embodiment of the present disclosure.
Fig. 4B is an example strain map of the amount of force applied to each sensor in the sensing array, according to one embodiment of the present disclosure.
Fig. 4C is an example weight map, according to one embodiment of the present disclosure.
Fig. 5 is a flow diagram depicting an exemplary process for measuring weight in a neonatal care system with a sensing array according to one embodiment of the present disclosure.
Fig. 6 is a flow diagram depicting an exemplary process for measuring weight in a neonatal care system with a sensing array according to one embodiment of the present disclosure.
Fig. 7A is a side view of an example sensing array, patient, extraneous object, load cell array, and mattress, according to one embodiment of the present disclosure.
Fig. 7B is a top view of the example sensing array, patient, extraneous objects, and mattress, according to one embodiment of the present disclosure.
Fig. 7C is an example strain map illustrating the strain indicated at each sensor in the sensing array according to one embodiment of the present disclosure.
Fig. 7D is an example weight map illustrating the weight indicated at each sensor in the sensing array according to one embodiment of the present disclosure.
Fig. 8 is a flow diagram depicting an exemplary process for measuring weight in a neonatal care system with a sensing array according to one embodiment of the present disclosure.
Fig. 9A is an example strain map indicating the weight for each sensor location in a neonatal care scale weighing system with a sensing array according to one embodiment of the present disclosure.
Fig. 9B is an example strain map indicating a modified weight for each sensor location in a neonatal care scale weighing system with a sensing array according to one embodiment of the present disclosure.
Fig. 10A is an example weight map indicating the weight for each sensor location in a neonatal care scale weighing system with a sensing array according to one embodiment of the present disclosure.
Fig. 10B is an example weight map indicating a modified weight for each sensor location in a neonatal care scale weighing system with a sensing array according to one embodiment of the present disclosure.
Fig. 11 is an exemplary scale manager for measuring weight in a neonatal care system with a sensing array according to one embodiment of the present disclosure.

### DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally, or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

The inventors have recognized a problem with current neonatal care systems, such as incubators, infant warmers, and other types of neonatal care systems and devices. As stated previously, neonatal care systems such as incubators and warmers may include integrated weighing systems to determine the weight of an infant in the system. Typically, a neonate's weight may be measured once a day to monitor growth and to determine the appropriate medication dosages and/or intravenous (IV) fluid intake. Currently, a healthcare provider may lift the neonate (with any attached equipment) off the mattress while ensuring that arms, legs, blankets, and clothing do not touch the mattress and affect the weight measurement. This lifting helps in baselining (i.e., taring) the measure of the scale. Once the scale is baselined, the healthcare provider may place the neonate back onto the mattress while all other equipment is held off. Hence, the scale may provide a measure of the neonate's weight. However, this process can interfere with the neonate's neurodevelopment as the lifting and other manipulation is a negative stimulus, can interfere with neonate's sleep, cause the neonate discomfort, and potentially cause a dangerous dislodgement of therapeutic tubes and/or sensors. Additionally, this lifting and measuring process may be somewhat cumbersome for the healthcare provider. Further, in contrast to a daily measurement, a more continuous measurement (e.g., once an hour) of the neonate's weight can more closely track the neonate's intake and output, and make it possible to determine an estimate of the neonate's metabolism. However, a continuous measurement may be more harmful and labor-intensive than daily measurement because of the lifting process involved with current systems.

In view of the foregoing problems and challenges recognized by the inventors through their extensive research and experience in the field of neonatal care systems, the inventors have developed the disclosed improved systems and methods for weighing an infant housed in an incubator, warmer, or other neonatal care system. More specifically, some embodiments of the present disclosure may use a tactile sensor array embedded in the mattress to enable the continuous measurement of the neonate's weight without disruption to the neonate's neurodevelopment, sleep, and comfort. Additionally, such embodiments may provide a strain map and/or a camera to isolate and/or distinguish the neonate's weight from the weight of other objects located on the mattress. In such embodiments, it may be possible to weigh the neonate without manual lifting, thus eliminating a negative stimulus to the neonate, a potential cause of tube dislodgement, and the cumbersome process of baselining and weighing. In these ways, such embodiments can provide continuous neonate weighing to enable quantification of inputs such as medication and intravenous fluids, and the quantification of outputs, e.g., waste. Further, such embodiments may determine the neonate's weight distribution, e.g., the weight of the neonate's head, versus the weight of the rest of the body, and the like. Additionally, such embodiments may make it possible to determine the metabolism of the neonate.

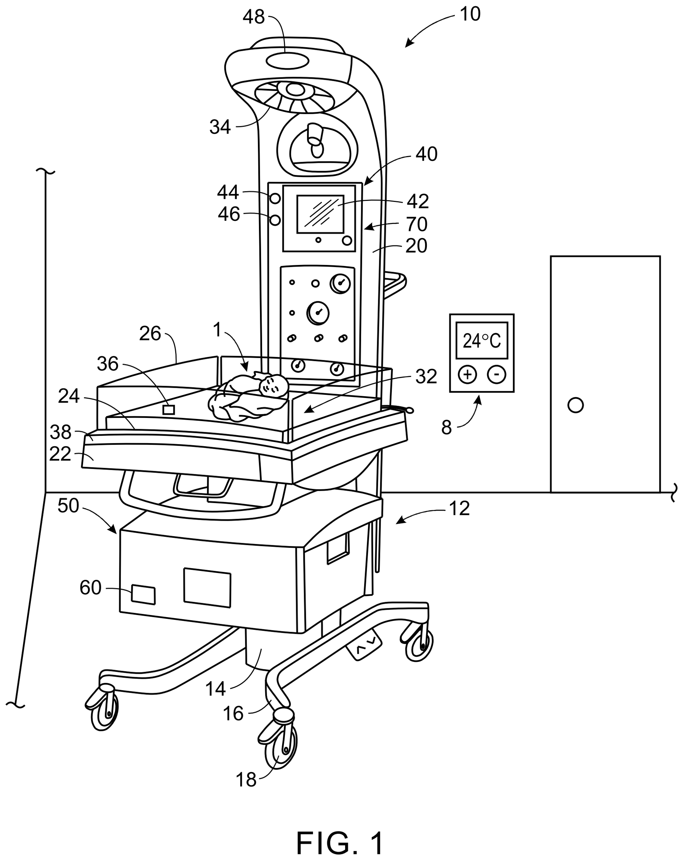

Fig. 1 is a perspective view of an exemplary neonatal care system 10 that measures weight with a sensing array according to one embodiment of the present disclosure. The neonatal care system 10 is shown within a room, such as a labor and delivery suite, or a neonatal intensive care unit, within a medical facility. The ambient air temperature within the room is controlled by room thermostat 8, which is adjustable up and down according to the specification of the patient and medical personnel in a customary manner.

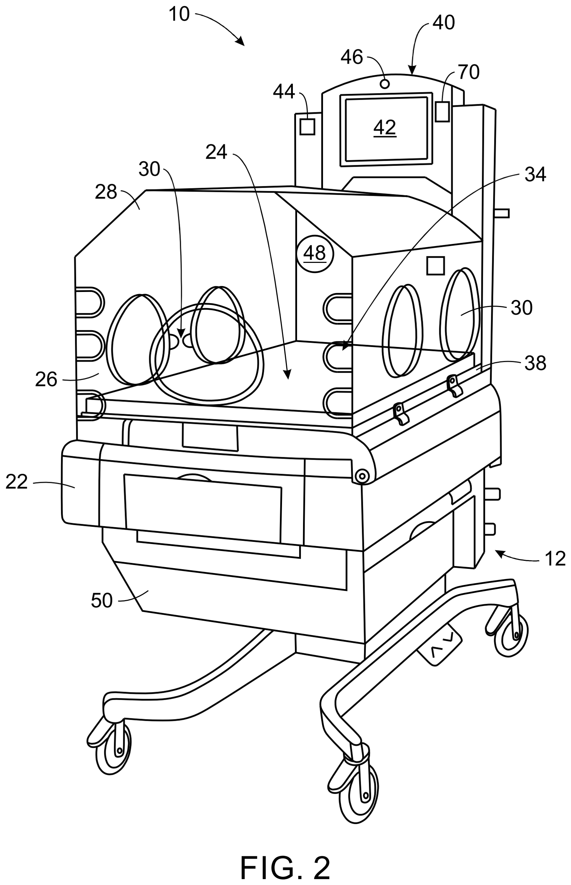

The neonatal care system 10 may define an infant warmer including a stand 12 supported by legs 14 and feet 16 provided with wheels 18 in a manner presently known in the art. The walls 26, and in the case of an incubator, a cover 28 (See Fig. 2), generally surround and cover the mattress 24, to prevent the patient 1 from falling from the mattress 24 and also to maintain a controlled environment within the interior. The air within the interior defined by the walls 26 (and when present, the cover) is also referred to as inside air 32. The heater 34, e.g., radiative heater, may be a heat generating device such as those used within the exemplary warmers described above. The patient 1 is warmed using the heater 34. Additionally, the stand 12 also supports an enclosure 50 (e.g., drawer for storage).

A column 20 extends upwardly from the stand 12. The column 20 may include a controller 70 (e.g., a microprocessor, computer processing circuit, and the like) for operating the neonatal care system 10 in a manner presently known in the art. The platform 22 may be supported by the base on the stand 12, and may be height adjustable along the column 20 in a manner presently known in the art. Further, the platform 22 is configured to support a sensing array 38, which may be incorporated (e.g., embedded) within and/or below the mattress 24, which is configured to support the patient 1. The sensing array 38 may include a tactile sensor array on a flexible base, such as a piezoresistive, piezoelectric, or a capacitive array, used as a touch sensor for tactile perception. In response to a tactile stimulus, the sensing array 38 may provide signals indicating information about forces at the points of contact. Further the sensing array 38 may be a flexible, stretchable, and thin material. According to some embodiments of the present disclosure, the array is a piezoresistive array that may be embedded within the mattress 24. Additionally, the controller 70 may include a scale manager, or a portion thereof, for performing weighing with the sensing array 38 as described herein. The sensing array 38 can measure a strain imposed on the array by anything and/or anyone located thereon. Additionally, the sensing array 38 may include a load cell array that is located below the sensing array 38. In some embodiments of the present disclosure, the load cell array may be embedded within the mattress 24. Alternatively, the load cell array may be located beneath the mattress 24. Each load cell of the load cell array may be located beneath one or more elements of the tactile sensor array. Further, the load cell array may be a set of force transducers, each of which may produce a signal that is indicative of the weight located thereon. Although the term, load cell, is used in the disclosure, it should be understood that the load cell array may incorporate any sensor(s) or device(s) that generates a signal that can be measured, and is representative of weight or force on each element of the load cell array.

The scale manager may periodically use the sensing array 38 to take a weight measurement of the patient 1 without lifting the patient 1 from the mattress 24. More specifically, the scale manager may identify a set of sensing array elements on which the patient's body is located. Additionally, the scale manager may determine the patient's weight by correlating the strain on each sensing array element to a measured weight, and summing the measured weights correlating to each sensing array element on which the patient's body is located. Alternatively, the scale manager may identify the load cells located beneath the sensing array elements on which the patient's body is located. In such a scenario, the scale manager may determine the patient weight by summing the weights measured by the identified load cells. In embodiments where the load cell array is located beneath the mattress, the mattress weight may show up as an increased weight value that is uniform across all the load cells. Accordingly, some embodiments of the present disclosure may subtract this uniform value from the measured weight.

As stated previously, the scale manager may take periodic weight measurements. In this way, the scale manager may generate a data feed of weight measurements. These periods can be one or more times a second, several seconds, one or more minutes, one or more hours, and the like. As such, it is possible that a healthcare provider, or other person, may place objects on (or remove objects from) the patient 1 and/or mattress 24, which may affect the measured weight. However, in order to determine the weight measurement more accurately, it may be useful to identify such objects and exclude the weight of these objects from the weight measurement. Accordingly, in one embodiment of the present disclosure, the scale manager may identify the location of such objects based on the output of the sensing array 38. Additionally, or alternatively, the neonatal care system 10 may include an image sensor 48. The image sensor 48 may capture individual images, and/or video, of the patient 1 and mattress 24. Further, the scale manager may analyze these images to identify where the patient 1 is located on the mattress 24, and thus, identify the elements of the sensing array 38 and/or load cell array that are useful for measuring the patient's weight.

The neonatal care system 10 further includes a user interface 40, which may include a display 42 configured to provide warning indications (text, colors, icons, and the like) as well as messages relating to operation of the neonatal care system 10. Additionally, the user interface 40 may include a speaker 44 and one or more lights 46. The speaker 44 and lights 46 may provide further information regarding the operational status of the neonatal care system 10.

Additionally, the speaker 44 and lights 46 may communicate information to a healthcare provider and/or operator via sounds, spoken text, spoken words, flashing, varying colors, and/or the lights being on or off. In this manner, as is discussed further below, the user interface 40 provides feedback customary of infant care systems 10 presently known in the art, but also additional information, warnings, and/or the like according to the present disclosure. It should be recognized that the user interface 40 may also or alternatively be provided via an external device (e.g., a mobile device such as a tablet or smart phone) in communication with the neonatal care system 10. For example, a smart phone may serve as the display 42, speaker 44, and/or lights 46 (alone or in conjunction with another display 42, speaker 44, and lights 46, on the neonatal care system 10) that communicates with the neonatal care system 10 via Bluetooth^{®} or another wireless protocol known in the art.

Fig. 2 is a perspective view of an exemplary neonatal care system 10 that measures weight with a sensing array 38 according to one embodiment of the present disclosure. In this example, the neonatal care system 10 is similar to that of Fig. 1, but as an incubator rather than an infant warmer. Similar to Fig. 1, the neonatal care system 10 of Fig. 2 includes stand 12, platform 22, mattress 24, walls 26, heater 34, sensing array 38, user interface 40, image sensor 48, enclosure 50, and controller 70. In the incubator, the patient 1 is warmed using warm air flowing in the incubator from the heater 34 and a fan (not shown) located below the mattress 24 and platform 22. Additionally, the neonatal care system 10 of Fig. 2 includes a cover 28, whereby the interior of the neonatal care system 10 is defined by the walls 26 and the cover 28. Further, the incubator of Fig. 2 includes portholes 30 within the walls 26 and/or cover 28 to provide access to the interior (e.g., patient 1, mattress 24, and/or the platform 22) without opening one or more of the walls 26 and/or the cover 28 in a manner presently known in the art. The image sensor 48 and controller 70 of Fig. 2 may be similar to the image sensor 48 and controller 70 of Fig. 1. As such, the controller 70 may include a scale manager that may perform weighing with the sensing array 38 and/or load cell array as described with respect to Fig. 1.

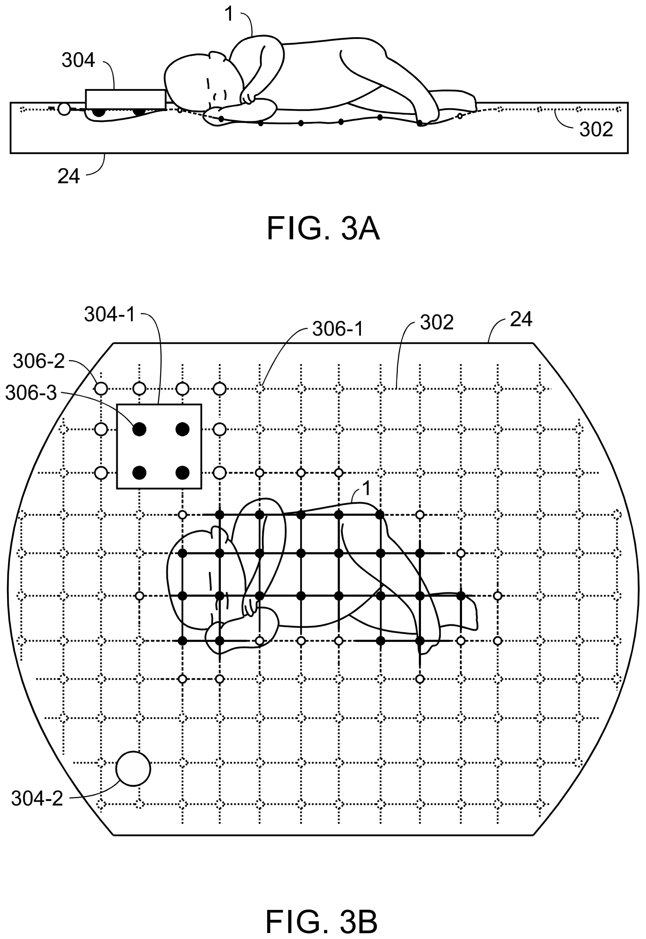

Fig. 3A is a side view of an example sensing array 302, patient 1, extraneous object 304, and mattress 24, according to one embodiment of the present disclosure. The sensing array 302 may be similar to the sensing array 38. In this example, the mattress 24 is deformed by the weight of the patient 1 and extraneous object 304 located thereon. Accordingly, the sensing array 302 may also deform with the mattress. However, the sensors of the sensing array 302 may or may not deform. Rather, the sensors respond to the forces and/or pressure of the patient 1 and extraneous object 304. For example, piezoelectric sensors may generate a voltage in response to the forces and/or pressure.

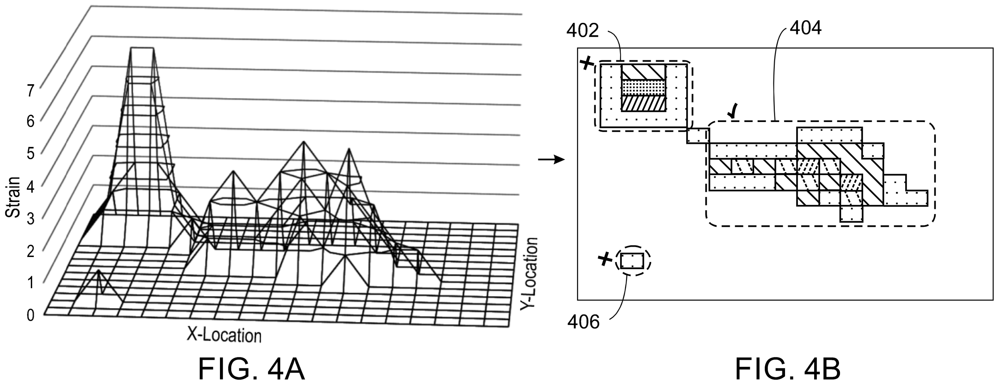

Fig. 3B is a top view of the example sensing array 302, patient 1, extraneous objects 304-1, 304-2, and mattress 24, according to one embodiment of the present disclosure. The example sensing array 302 may include multiple piezoelectric sensors 306 arranged in a grid pattern. According to some embodiments of the present disclosure, each of the piezoelectric sensors 306 may produce an electric current in response to the amount of strain due to the weight of the portion of the extraneous object 304 and patient 1 that falls on that sensor location on the mattress 24. In this example, the pattern of the sensors' outline represents the amount of force applied to the respective sensor 306. More specifically, the sensors 306-1 represent no force; the sensors 306-2 represent a lower amount of force; and, the sensors 306-3 represent a larger amount of force. For the purpose of clarity, in this figure, the terms lower and larger are used to describe the force here. However, the amount of force is described in greater detail with respect to Figs. 4A and 4B.

Fig. 4A is an example strain map 400A due to the amount of force applied to each sensor 306 in the sensing array 302, according to one embodiment of the present disclosure. According to some embodiments of the present disclosure, the scale manager may generate the strain map 400A based on the outputs of the sensing array 302. The strain map 400A may be a graphical representation of the strain of the sensors 306 which depends on the force applied by the elements located on the mattress 24. In this example, the strain map 400A is represented as a three-dimensional (3D) graph, where the X and Y dimensions represent the locations of the sensors 306 in the X and Y dimensions of the sensing array 302. Additionally, the Z dimension represents the amount of strain of the sensor 306 at each X, Y location. For clarity, this example strain map 400A represents strain generically, without reference to specific units. Further, in this example, the lines of the strain map 400A represent the slopes between sensors 306 having different values of strain. As shown, the extraneous object 304-1 appears to give rise to the relatively greatest levels of strain, the patient 1 creates relatively lower levels of strain, and extraneous object 304-2 cerates the relatively lowest levels of strain. In this way, the strain map 400A may indicate the presence of the three elements on the mattress 24: the patient 1 and extraneous objects 304-1, 304-2.

Fig. 4B is an example strain map 400B representative of the strain indicated in strain map 400A, according to one embodiment of the present disclosure. In this example, the strain map 400B is represented as a two-dimensional (2D) mapping, wherein each different level of strain of the sensors 306 is represented graphically at an X, Y location that corresponds to the X, Y location of the sensor 306 in the sensing array 302. Further, the graphic representation indicates greater levels of strain with greater levels of hashing at each location. Hence, the locations with no hatching represent no strain, and the locations with the greatest number of hatch lines represent the greatest relative strain. According to some embodiments of the present disclosure, the scale manager may determine outlines of elements located on the mattress 24 based on the strain map 400A or 400B. Accordingly, the strain map 400B includes outlines for each of the patient 1, and extraneous objects 304-1, 304-2.

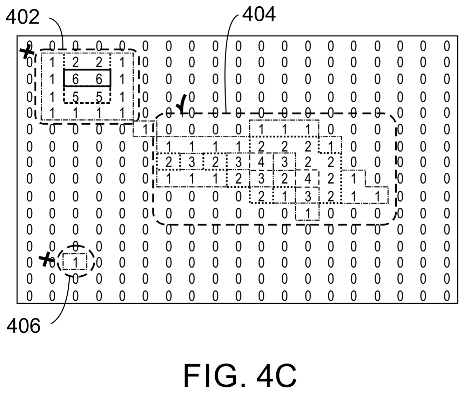

Fig. 4C is an example weight map 400C, according to one embodiment of the present disclosure. In this example, the weight map 400C is represented as a two-dimensional (2D) mapping, wherein the weight value corresponding to the strain level at each of the sensors 306 is represented numerically at an X, Y location that corresponds to the X, Y location of the sensor 306 in the sensing array 302. The numeric value may indicate a weight in grams, ounces, or other units of weight measure depending on the embodiment implemented. According to some embodiments of the present disclosure, the scale manager may generate the weight map 400C based on strain map 400A or strain map 400B. More specifically, the scale manager may determine the weight based on a strain-weight model that correlates the strain at a sensor 306 with a weight value. As stated previously, the scale manager may determine outlines of elements located on the mattress 24 based on the strain map 400A and/or 400B. Accordingly, the strain map 400B includes outlines for each of the patient 1, and extraneous objects 304-1, 304-2. Hence, the scale manager may determine the patient weight may by adding the weights indicated at the sensors within the outline for the patient 1. In these ways, some embodiments of the present disclosure may determine the weight of the patient 1 on a continuous basis (e.g., every hour) without lifting the patient 1, and thus avoiding the negative effects of lifting on neurodevelopment, sleep, and patient comfort.

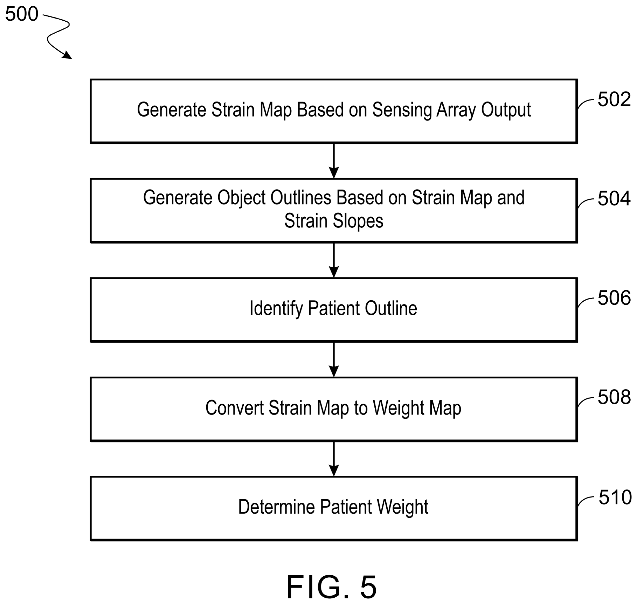

Fig. 5 is a flow diagram depicting an exemplary process 500 for measuring weight in a neonatal care system with a sensing array according to one embodiment of the present disclosure. The scale manager may perform the process 500 to measure the weight of the patient on a continuous basis, and without disturbing the patient 1.

At operation 502, the scale manager may generate a strain map based on the output of the sensing array 302. As stated previously, the sensing array 302 may include a piezoresistive tactile sensor array that is embedded within the mattress 24 of a neonatal care system 10. Generating the strain map may involve generating a graphical representation of strain. This graphical representation may include a series of sloping lines between neighboring points in 3D space. The X, Y locations of these points may represent the X, Y locations of the sensors 306 of the sensing array. Thus, the Z coordinate may represent the amount of strain indicated by the electric current generated by the sensor 306 at the X, Y location. Alternatively, generating the strain map (e.g., strain map 400B) may involve populating a two-dimensional array with numeric strain values, where the location of each element of the array corresponds to the X, Y location of the sensor 306 having the corresponding strain value.

At operation 504, the scale manager may generate the object outlines based on the strain levels and strain slope. More specifically, the scale manager may determine outlines of the elements on the mattress by identifying contiguous sets of sensors 306 for each of the objects based on strain levels and strain slope. According to some embodiments of the present disclosure, the scale manager may use the sensors 306 associated with each of the high strain points as the starting points for each object. The high strain points may be the locations in the sensing array where the sensors 306 sense the relatively highest strain values within a contiguous set of sensors 306. For example, in the example strain map 400B, described with respect to Fig. 4B, the highest strain points are the locations within the extraneous object 304-1, indicated by the heaviest hatching. Hence, the scale manager may identify the extraneous object 304-1 by determining the contiguous points with the high strain point. Further, the high strain point for the patient is also indicated by the locations in the example strain map 400B with the relatively heaviest hatching. Accordingly, the scale manager may identify these locations as high strain points. Similar to identifying the extraneous object 304-1, the scale manager may identify the contiguous points with the patient's high strain point, and thus identify the outline of the patient 1. The scale manager may similarly identify the outline of extraneous object 304-2. Accordingly, the scale manager may determine the weight of each using the strains indicated in the corresponding locations of the strain map and the strain-weight model.

At operation 506, the scale manager may identify the patient 1 from the identified objects. According to some embodiments of the present disclosure, the scale manager may identify the patient based on the strain levels (e.g., weight), outline size (e.g., area), and strain slope. For example, objects 304-1 and 304-2 occupy a relatively small area, e.g., below a predetermined threshold for the area the patient 1 occupies, and thus, the scale manager may determine that the extraneous objects 304-1, 304-2, are not the patient 1. For example, the scale manager may calculate the predetermined threshold for the area occupied by the patient 1 based on a measurement of the patient's length and width, potentially measured by a healthcare provider. Alternatively, or additionally, the scale manager may compare the weight of the extraneous objects 304-1, 304-2 to a threshold weight (e.g., a previous weight measurement) for the patient 1, and determine that the extraneous objects 304-1, 304-2, are not the patient 1. For example, the scale manager may determine that the weight of any object that exceeds a predetermined threshold change from a previous measurement, is too light (or heavy) to be the patient 1, and thus, is not the patient 1. Alternatively, the scale manager may determine a density of the patient based on the previous weight and size measurements. Accordingly, the scale manager may determine a high weight threshold from the patient 1 that can be applied to each sensor 306. Hence, the scale manager may exclude any object with a weight exceeding this density from being considered as being the patient 1. Additionally, or alternatively, the scale manager may compare the strain slope of each identified object to a predetermined strain slope threshold to identify the patient 1. More specifically, the relative strain sensed by each of the sensors 306 under the patient may not exceed the predetermined threshold strain slope. The strain slope refers to the difference in detected strain between contiguous sensors 306. For example, the weight of a dense object may merely fall on the sensors 306 on which the dense object is located. Thus, the neighboring sensors may detect no strain. In such a case, the dense object may have a high strain slope (potentially infinite) at the edges, and a relatively low (potentially zero) strain slope elsewhere within the object outline. Accordingly, to determine the threshold strain slope, the scale manager may use the high weight threshold to determine a high strain slope threshold at the outline of the patient 1. Thus, any strain slope exceeding the threshold strain slope may not indicate the location of the patient 1. In these ways, the scale manager may determine that an identified object is not the patient 1 because the strain slope between the set of sensors 306 associated with the object exceeds a predetermined threshold, the strain exceeds a predetermined threshold, the weight exceeds a predetermined threshold, and/or the size of the object (indicated by the set of identified sensors 306) is less than a predetermined threshold.

At operation 508, the scale manager may generate a weight map, based on the strain map, e.g., strain map 400A, 400B, and a strain-weight model. As stated previously, the strain map may indicate the amount of strain of (e.g., due to force applied) at each sensor 306 of the sensing array 302. Further, the strain-weight model may correlate an amount of strain with a weight. More specifically, the strain-weight model may be a strain-weight calibration that is specific to the sensors 306. Alternatively, the scale manager, or another process, may generate the strain-weight model experimentally for the use scenario. For example, an operator may place calibrated weights on the mattress 24, and configure the scale manager to correlate the detected strain to the calibrated weight. In another alternative, the strain-weight model may be a look-up table or a functional equation.

Accordingly, the scale manager may generate a 2D table where each of the sensors 306 is represented at an X, Y location that corresponds to the X, Y location of the sensor 306 in the sensing array 302. Further, the scale manager may determine the strain from each of the sensors 306, and populate each of the table locations with a numerical weight based on the weight corresponding to the strain according to the strain-weight model. According to some embodiments of the present disclosure, the scale manager may generate the weight map before identifying the patient 1.

At operation 510, the scale manager may determine the weight of the patient 1. Determining the weight of the patient may involve summing the weight corresponding to all the sensors 306 that the scale manager identifies as being strained by the patient's weight.

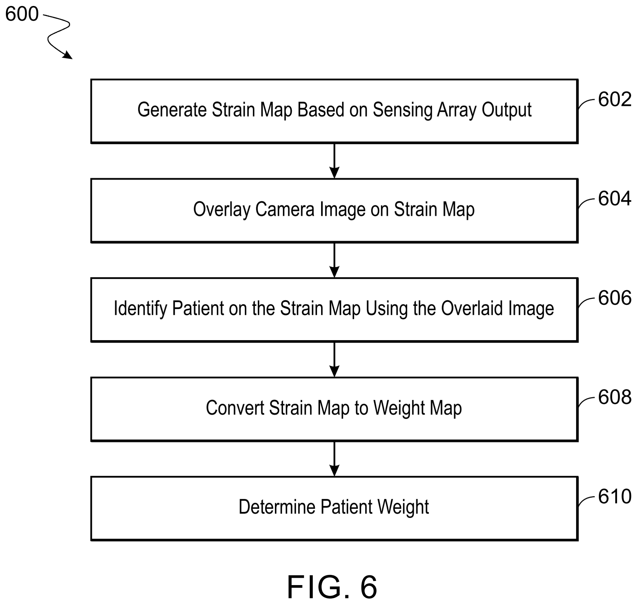

Fig. 6 is a flow diagram depicting an exemplary process 600 for measuring weight in a neonatal care system with a sensing array according to one embodiment of the present disclosure. The scale manager may perform the process 600 to measure the weight of the patient on a continuous basis, and without disturbing the patient 1.

At operation 602, the scale manager may generate a strain map based on the output of the sensing array 302. As stated previously, the sensing array 302 may include a piezoresistive tactile sensor array that is embedded within the mattress of a neonatal care system. Accordingly, the scale manager may use the sensor values from the sensing array 302 to populate a two-dimensional table where each element in the table includes the sensor value of the sensor 306 at a location corresponding to the location in the table.

At operation 604, the scale manager may overlay a captured image on a strain map (e.g., strain map 400A). As stated previously, the image sensor 48 may capture an image of the patient 1, extraneous objects 304, and the mattress 24. Thus, overlaying the captured image on the strain map 400A may involve correlating the orientation of the strain map with the orientation of the captured image.

At operation 606, the scale manager may identify the patient 1 on the strain map 400A using the overlaid image. Identifying the patient 1 may involve identifying the sensors 306 on which the patient 1 is located in the overlaid image. According to some embodiments of the present disclosure, identifying the patient may involve the use of a machine learning model trained to identify images of infants. Thus, the scale manager may use a recurrent neural network to identify the body of the patient in the image. However, other machine learning models may also, or alternatively, be used, such as, convolutional neural network (CNN), you only look once (YOLO), support vector machines (SVM), and the like. Further, the scale manager may identify the sensors 306 beneath the patient by using the image overlay to determine which of the sensors 306 overlap with the portions of the image where the patient is located.

At operation 608, the scale manager may generate a weight map based on the strain map, e.g., strain map 400A/ 400B and a strain-weight model. As stated previously, the strain map 400A may indicate the amount of strain of (e.g., due to force applied on) each sensor 306 of the sensing array 302. Further, the strain-weight model may correlate an amount of strain with a weight. Accordingly, the scale manager may generate a 2D table where each of the sensors 306 is represented at an X, Y location that corresponds to the X, Y location of the sensor 306 in the sensing array 302. Further, the scale manager may determine the strain from each of the sensors 306, and populate each of the table locations with a numerical weight based on the weight corresponding to the strain according to the strain-weight model.

At operation 610, the scale manager may determine the weight of the patient 1. Determining the weight of the patient 1 may involve identifying the weights in the weight map associated with sensors 306 identified in the overlaid image. Additionally, determining the weight of the patient 1 may involve summing the identified weights.

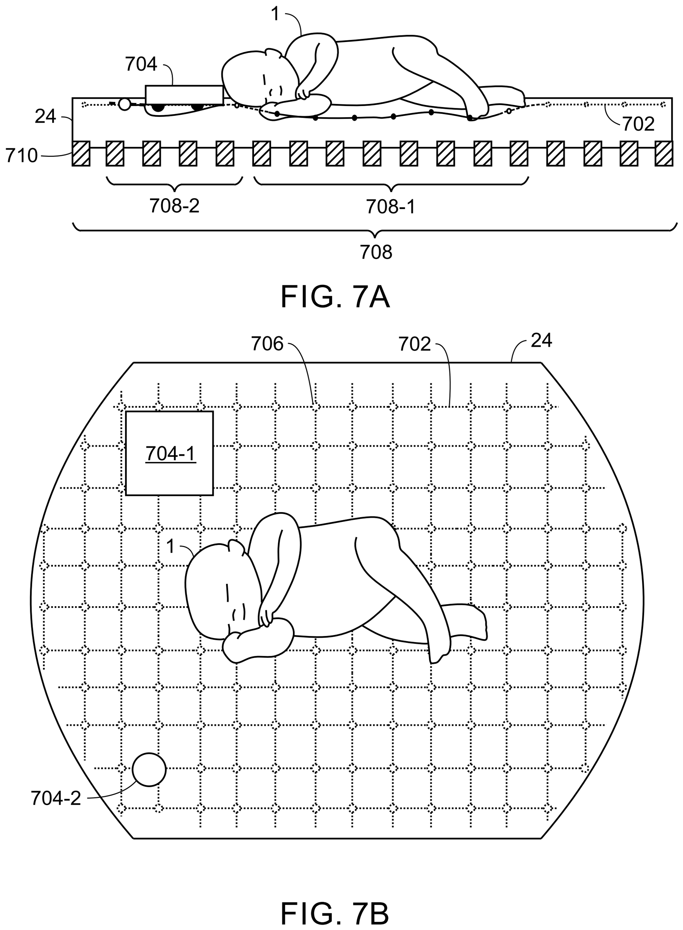

Fig. 7A is a side view of an example sensing array 702, patient 1, extraneous object 704, load cell array 708, and mattress 24, according to one embodiment of the present disclosure. The sensing array 702 may be similar to the sensing array 38. Further, the load cell array 708 may include multiple load cells 710, where a load cell 710 may be positioned beneath each sensor of the sensing array 702. In this example, the load cell array 708 is located beneath the mattress 24.

Fig. 7B is a top view of the example sensing array 702, patient 1, extraneous objects 704-1, 704-2, and mattress 24, according to one embodiment of the present disclosure. The example sensing array 702 may include multiple piezoelectric sensors 706 arranged in a grid pattern. According to some embodiments of the present disclosure, each of the piezoelectric sensors 706 may produce an electric current in response to the amount of force applied from the weight of the portion of the extraneous object 704 and patient 1 located on the mattress 24. Additionally, a load cell 708 (not shown) may be positioned below each of the sensors 706. Alternatively, there may be fewer load cells 708 than strain sensors 706. As such, each load cell 708 may be positioned beneath multiple strain sensors 706. Accordingly, each load cell 708 may measure weight based on the strain sensed by the load cell's corresponding set of sensors. Further, while the load cells 708 in this example are located beneath the mattress 24, as stated previously, the load cells 708 may, alternatively, be embedded within the mattress 24.

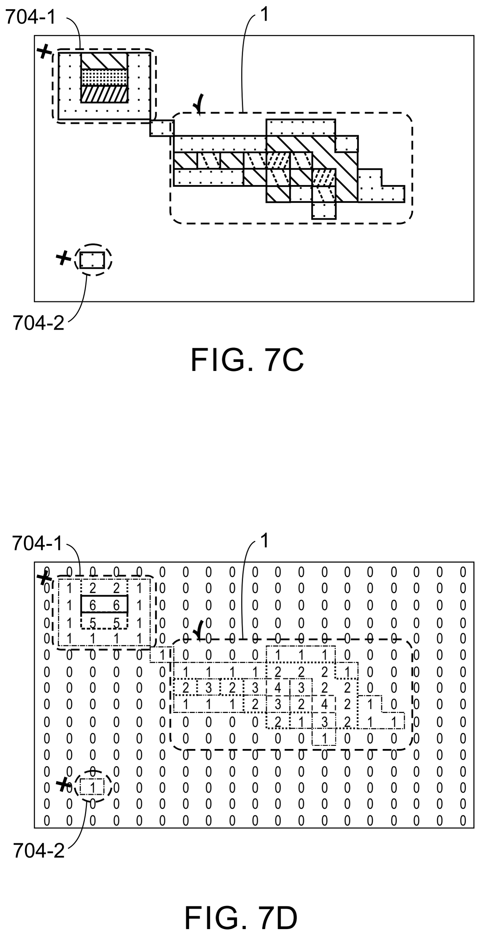

Fig. 7C is an example strain map 700C illustrating the strain indicated at each sensor 706 in the sensing array 702 according to one embodiment of the present disclosure. The strain map 700C may be similar to the strain map 400B described with respect to Fig. 4B. As stated previously, the scale manager may identify the sensors 706 positioned beneath the patient, as described with respect to Figs. 5, 6. Accordingly, the strain map 700C includes outlines around the strain indicators for the sensors 706 under each of the patient 1 and extraneous objects 704-1, 704-2.

Fig. 7D is an example weight map 700D illustrating the weight indicated at each sensor 706 in the sensing array 702 according to one embodiment of the present disclosure. The weight map 700D may be similar to the weight map 400C, described with respect to Fig. 4C. However, in contrast to the generation of the weight map 400C using a strain-weight model, the scale manager may generate the weight map 700D by using the weights measured by the load cells 708 under the sensors 706. Similar to the strain map 700C, the weight map 700D includes outlines around the weight values for the load cells 708 under each of the patient 1 and extraneous objects 704-1, 704-2. Accordingly, as described with respect to Figs. 5, 6, the scale manager may use the outlines to identify the load cells 708 located beneath the patient 1. Thus, according to some embodiments of the present disclosure, the scale manager may determine the weight of the patient by summing the weights from the load cells 708-1 located under the patient, and disregard the weights from the load cells 708-2 located under the extraneous objects 704-1, 704-2.

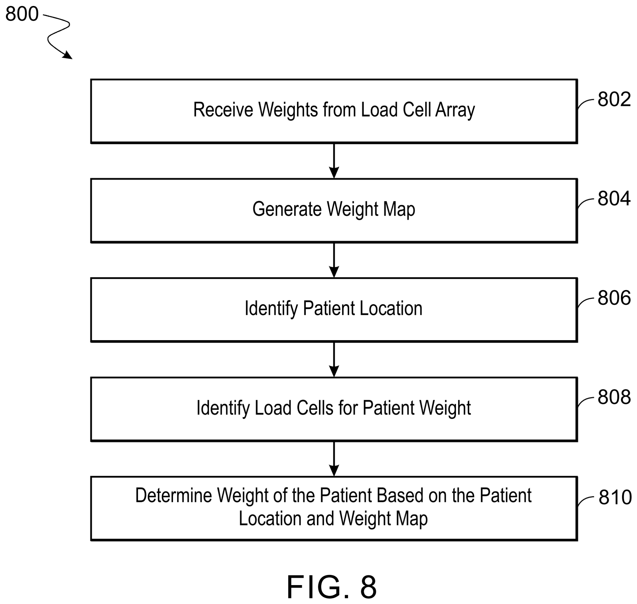

Fig. 8 is a flow diagram depicting an exemplary process 800 for measuring weight in a neonatal care system with a sensing array according to one embodiment of the present disclosure. The scale manager may perform the process 800 to measure the weight of the patient on a continuous basis, and without disturbing the patient 1.

At operation 802, the scale manager may receive weight from a load cell array, such as the load cells 708, described with respect to Figs. 7A through 7D. Each of the load cells 708 in the load cell array may measure a portion of the force on the mattress 24, including force caused by the weight of the patient 1 and extraneous objects (e.g., extraneous objects 704-1, 704-2).

At operation 804, the scale manager may generate a weight map based on the received weights from the load cells 708. According to some embodiments of the present disclosure, the weight map (e.g., weight map 700D) may be a two-dimensional array that includes a numeric value for the weight measured by each of the load cells 708. The two-dimensional array may correlate the weight measures with the location of the load cells 708 with respect to the mattress 24.

At operation 806, the scale manager may identify the patient location. Identifying the location of the patient may involve identifying which load cells can provide measures of the patient's weight. Similar to identifying the sensors 306 on which the patient 1 is located (as described with respect to Figs. 5 and 6), the scale manager may identify the load cells on which the patient is located by capturing an image of the patient 1 on the mattress 24, identifying the patient 1 within the image, and identifying the load cells 708 that overlap with the location of the patient in an overlaid image. According to some embodiments of the present disclosure, identifying the patient may involve the use of a recurrent neural network trained to identify images of infants. Thus, the scale manager may use the recurrent neural network to identify the body of the patient in the image. Further, the scale manager may identify the load cells 708 beneath the patient by using the image overlay to determine which of the load cells 708 overlap with the portions of the image where the patient is located.

Alternatively, the scale manager may identify the patient location using an image overlay with the sensing array 702. More specifically, the scale manager may generate a strain map (e.g., strain map 700C) based on the strain values provided by the sensing array 702. Additionally, the scale manager may identify the sensors 706 on which the patient is located using the captured image described above, to identify where the patient location overlaps with the sensors 706 in the image overlaid on the strain map 700C. Further, the scale manager may identify which of the load cells 708 correspond to the identified sensors 706. As stated previously, the load cells 708 may be positioned beneath each sensor 706. Alternatively, there may be fewer load cells 708 than sensors 706. As such, each load cell 708 may way the portion of the patient 1 corresponding to the location of multiple sensors 706.

At operation 808, the scale manager may calculate the patient's weight based on the weight map 700D and the patient location. More specifically, the scale manager may sum the measured weights from the load cells 708 that are identified as corresponding to the patient's location as described above.

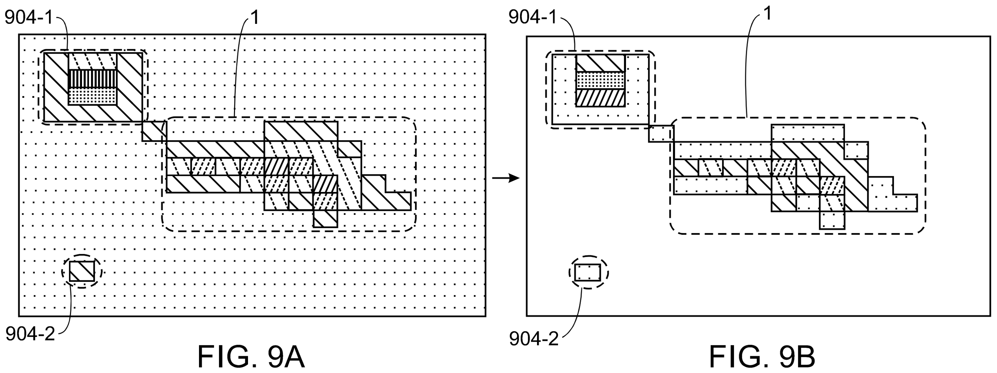

Fig. 9A is an example strain map 900A indicating the strain for each sensor location in a neonatal care scale weighing system with a sensing array according to one embodiment of the present disclosure. In this example, the strain map 900A indicates the presence of an additional uniform strain on the sensing array. More specifically, there no zero strain values in the strain map 900A. Rather, all of the sensors 306 indicate a level of strain, which may indicate that an extraneous object is located on the mattress 24, adding a uniform strain value that the sensors detect. For example, if a healthcare professional places a second mattress on the mattress 24, the second mattress may add a uniform strain detected by the sensor array. Thus, according to some embodiments of the present disclosure, the scale manager may determine that the sensing array 302 is detecting a uniform strain by determining that the strain map 900A has no zero values. Alternatively, the scale manager may use a threshold strain value to determine uniform strain. More specifically, if there are no strain values lesser than the threshold strain value, the scale manager may determine there is a uniform strain. Further, according to some embodiments of the present disclosure, the scale manager may plot a histogram of sensed strain values. The plotted histogram may indicate a minimum strain value. Hence, if the strain map 900A has no strain values lesser thana the minimum value, the scale manager may determine there is a uniform strain.

Fig. 9B is an example strain map 900B indicating a modified strain for each sensor location in a neonatal care scale weighing system with a sensing array according to one embodiment of the present disclosure. In this example, the strain map 900B represents the result of the scale manager modifying the strain map 900A by removing (e.g., subtracting) the uniform strain from all strain values. In some embodiments, the scale manager may determine a uniform weight corresponding to the uniform strain using the strain-weight model. Accordingly, the scale manager may subtract the uniform weight from the weight map for the strain map 900A.

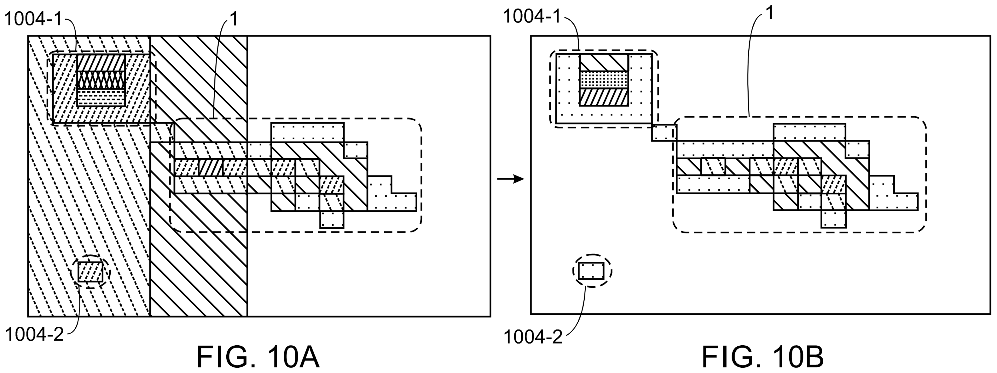

Fig. 10A is an example strain map 1000A indicating the strain for each sensor location in a neonatal care scale weighing system with a sensing array according to one embodiment of the present disclosure. In this example, the strain map 1000A represents a scenario where a healthcare provider has added a wedge to the mattress to prop up the patient's head. Accordingly, the strain map indicates a presence of an additional gradient strain on the strain sensor array. In other words, the strain map 1000A indicates a sloping increase in strain values from right to left. Accordingly, the scale manager may identify the sensors showing the gradient strain increases, and subtract the gradient strain increases from the sloping subset of strains. According to some embodiments, the scale manager may identify the sloping increase or decrease and make a correcting modification using standard slope detection and correction methods. In one example, the scale manager may average the strain detected in each column (or row) of the sensing array, and identify a sloping increase based on continuous change in average slope across columns (or rows). Alternatively, the scale manager may identify an additional gradient weight in the weight map generated from the strain map 1000A. In such embodiments, the scale manager may identify the sloping increase or decrease in weight values, and make a correcting modification using standard slope detection and correction methods.

Fig. 10B is an example strain map 1000B indicating a modified strain for each sensor location in a neonatal care scale weighing system with a sensing array according to one embodiment of the present disclosure. In this example, the scale manager has modified the strain map 1000A by subtracting the sloping increase in strain amounts.

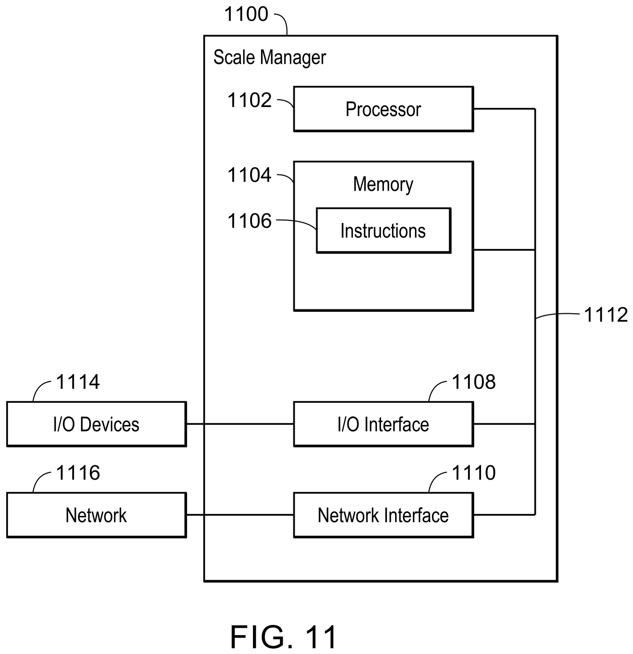

Fig. 11 is an exemplary scale manager 1100 for measuring weight in a neonatal care system with a sensing array according to one embodiment of the present disclosure. The example scale manager 1100 may measure weight with a sensing array, as described with respect to Figs. 1, 2, 3A, 3B, 4A, 4B, 4C, 5, 6, 7A, 7B, 7C, 7D, 8, 9A, 9B, 10A, 10B, and 11. In this example, the scale manager 1100 includes a processor 1102, memory 1104, input-output (I/O) interface 1110, and network interface 1112, which may be connected by an interconnect 1114. The processor 1102 may be a computer processing circuit (e.g., a central processing unit (CPU)) that retrieves and executes programming instructions 1106 stored in the memory 1104 to perform the functionality described herein. The interconnect 1114 may move data, such as programming instructions, between the processor 1102, memory 1104, I/O interface 1110, and network interface 1112. The interconnect 1114 may include one or more buses.

The memory 1104 may be a computer memory or storage device, including volatile memory, such as a random access memory (RAM) device (e.g., static RAM, dynamic RAM, and the like), non-volatile memory, such as a hard disk drive, solid state device (SSD), removable memory cards, optical storage, flash memory devices, and the like. In some examples, the memory 1104 may include volatile and non-volatile memory devices. Further, the memory 1104 may store instructions 1106 for measuring weight with a sensing array as described with respect to Figs. 1, 2, 3A, 3B, 4A, 4B, 4C, 5, 6, 7A, 7B, 7C, 7D, 8, 9A, 9B, 10A, 10B, and 11.

Additionally, the scale manager 1100 may be in electronic communication with I/O devices 1116 through the I/O interface 1110, and with a network 1118 through the network interface 1112. The I/O devices 1116 may capture inputs and provide outputs as described herein. More specifically, the sensing array 38 and image sensor 48, described with respect to Figs. 1 and 2, and load cell array 708 described with respect to Figs. 7A, 7B, 7C, and 7D, may be input devices. Additionally, the output devices may include the display 42, speaker 44, and lights 46 described with respect to Figs. 1 and 2. The network 1118 may be an electronic communication network, such as a local area network, wide area network, and the like, for processing communications with the scale manager 1100. In some examples, the network 1118 may be wired, wireless (e.g., wi-fi, Bluetooth, or cellular), or some other computer communication network.

In some embodiments, the scale manager 1100 may be a server computer or similar device without a user interface but which receives requests from other computer systems having one or more user interfaces. Further, in some embodiments, the scale manager 1100 may be a portable computer, laptop, tablet computer, pocket computer, telephone, smart phone, or the like.

An example neonatal care system includes a processor and a memory. The memory includes instructions configured to cause the processor to receive strain amounts corresponding the strain sensors of a tactile sensor array that is disposed beneath a patient that is disposed on a mattress. Additionally, the instructions cause the processor to generate a strain map comprising a visualization of the strain amounts in association with the corresponding X, Y coordinate locations of the strain sensors. Further, the instructions cause the processor to generate a weight map based on the strain map and a strain-weight model. Additionally, the weight map includes weights corresponding to the strain amounts. Further, the instructions cause the processor to identify a patient location. Additionally, the instructions cause the processor to determine a weight of the patient based on the patient location and the weight map.

In one example, the instructions are executable by the processor to determine that the weight map indicates a presence of an additional uniform strain on the strain sensor array, determine an amount of uniform weight corresponding to the uniform strain, and subtract the uniform weight from each of the weights.

In one example, the instructions are executable by the processor to determine that the weight map indicates a presence of an additional gradient strain on the strain sensor array, determine gradient strain increases associated with a sloping subset of the strains, subtract the gradient strain increases from the sloping subset of strains, and generate the weight map based on a strain map resulting from subtracting the gradient strain increases.

In one example, the strain sensors include piezoelectric tactile sensors.

In one example, identifying the patient location includes identifying a contiguous set of strain values that correspond to a predetermined threshold that is indicative of a strain that the patient places on corresponding strain sensors.

In one example, determining that the predetermined threshold is indicative of the strain includes determining a density of the patient, determining a weight value threshold for a sensor based on the density and a number of sensors on which the patient is located, and determining that each of the weights corresponding to the contiguous set of strain values is less than or equal to the weight value threshold.

In one example, determining the weight of the patient includes summing a subset of weights of the weight map that are associated with a subset of strain sensors corresponding to the patient location.

In one example, identifying the patient location includes capturing an image of the patient on the mattress, identifying the patient within the image, overlaying the image on the strain map, and identifying a subset of strain sensors corresponding to where the patient and the subset of strain sensors overlap.

An example method includes receiving weights corresponding to load cells of a load cell array that is disposed beneath a patient that is disposed on a mattress. Additionally, the method includes identifying a patient location. Further, the method includes generating a weight map having the weights. Additionally, the method includes determining a weight of the patient based on the patient location and the weight map.

In one example, the method includes determining that the weight map indicates a presence of an additional uniform weight on the load cell array. Additionally, the method includes determining an amount of the additional uniform weight. Further, the method includes subtracting the uniform weight from the weights of the weight map.

In one example, the method includes determining that the weight map indicates a presence of an additional uniform weight on the load cell array. Additionally, the method includes determining an amount of the additional uniform weight. Further, the method includes subtracting the uniform weight from each of the weights.

In one example, identifying the patient location includes capturing an image of the patient on a mattress. Further, identifying the patient location includes identifying an image location of the patient within the image. Additionally, identifying the patient location includes overlaying the image on the weight map. Further, identifying the patient location includes identifying a subset of the load cells that overlap with the image location of the patient in the overlaid image.

In one example, identifying the patient location includes receiving strain amounts corresponding to strain sensors in a strain sensor array. Additionally, identifying the patient location includes generating a strain map comprising strain amounts associated with corresponding X, Y coordinate locations for the strain sensors. Further, identifying the patient location includes identifying a patient location based on the strain map, and identifying a subset of the load cells that correspond to a subset of the strain sensors that overlap with the patient location.

In one example, identifying the patient location comprises identifying a contiguous set of strain values in the strain map that correspond to a predetermined threshold that is indicative of a strain that the patient places on a corresponding plurality of strain sensors.

In one example, determining the weight of the patient includes summing a subset of the weights associated with a subset of strain sensors corresponding to the patient location.

An example computer-readable storage medium includes instructions executable by a processor to receive strain amounts corresponding to strain sensors in a strain sensor array that is disposed beneath a patient that is disposed on a mattress. Additionally, the instructions are executable by the processor to generate a strain map having strain amounts associated with corresponding X, Y coordinate locations for the strain sensors. Further, the instructions are executable by the processor to generate a weight map based on the strain map and a strain-weight model, the weight map having weights corresponding to the strain amounts. Additionally, the instructions are executable by the processor to identify a patient location. Further, the instructions are executable by the processor to determine a weight of the patient based on the patient location and the weight map.

In one example, the instructions are executable by the processor to determine that the weight map indicates a presence of an additional uniform weight on the strain sensor array, determine an amount of the additional uniform weight, and subtract the additional uniform weight from each of the weights of the weight map.

In one example, the instructions are executable by the processor to determine that the weight map indicates a presence of an additional gradient strain on the strain sensor array, determine gradient weight increases associated with a sloping subset of the weights, and subtract the gradient weight increases from the sloping subset of the weights.

In one example, the strain sensors include piezoelectric tactile sensors.

In one example, identifying the patient location includes identifying a contiguous set of strain values that correspond to a predetermined threshold that is indicative of a strain that the patient places on corresponding strain sensors.

As used herein, the term, mechanism, can encompass hardware, software, firmware, or any suitable combination thereof. In some embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A neonatal care system, comprising:
a processor; and
a memory comprising instructions configured to cause the processor to:
receive a plurality of strain amounts corresponding to a plurality of strain sensors in a strain sensor array that is disposed beneath a patient that is disposed on a mattress;
generate a strain map comprising a plurality of strain amounts associated with a corresponding plurality of X, Y coordinate locations for the plurality of strain sensors;
generate a weight map based on the strain map and a strain-weight model, the weight map comprising a plurality of weights corresponding to the plurality of strain amounts;
identify a patient location of a patient; and
determine a weight of a patient based on the patient location and the weight map.

2. The neonatal care system of claim 1, wherein the instructions are executable by the processor to:
determine that the weight map indicates a presence of an additional uniform weight on the strain sensor array;
determine an amount of the additional uniform weight; and
subtract the additional uniform weight from each of the plurality of weights of the weight map.

3. The neonatal care system of claim 1, wherein the instructions are executable by the processor to:
determine that the strain map indicates a presence of an additional gradient strain on the strain sensor array;
determine a plurality of gradient strain increases associated with a sloping subset of the plurality of strains;
subtract the plurality of gradient strain increases from the sloping subset of the plurality of strains; and
generate the weight map based on a strain map resulting from subtracting the plurality of gradient strain increases.

4. The neonatal care system of claim 1, wherein the strain sensors comprise piezoelectric tactile sensors.

5. The neonatal care system of claim 1, wherein identifying the patient location comprises identifying a contiguous set of strain values that correspond to a predetermined threshold that is indicative of a strain that the patient places on a corresponding plurality of strain sensors.

6. The neonatal care system of claim 5, wherein determining the predetermined threshold is indicative of the strain comprises:
determining a density of the patient;
determining a weight value threshold for a sensor based on the density and a number of sensors on which the patient is located; and
determining that each of a plurality of weights corresponding to the contiguous set of strain values is less than or equal to the weight value threshold.

7. The neonatal care system of claim 1, wherein determining the weight of the patient comprises summing a subset of a plurality of weights of the weight map, the plurality of weights being associated with a subset of strain sensors corresponding to the patient location.

8. The neonatal care system of claim 1, wherein identifying the patient location comprises:
capturing an image of the patient on the mattress;
identifying the patient within the image;
overlaying the image on the strain map; and
identifying a subset of the plurality of strain sensors corresponding to where the patient and the subset of the plurality of strain sensors overlap.

9. A method, comprising:
receiving a plurality of weights corresponding to a plurality of load cells of a load cell array that is disposed beneath a patient that is disposed on a mattress;
identifying a patient location;
generating a weight map comprising the plurality of weights; and
determining a weight of the patient based on the patient location and the weight map.

10. The method of claim 9, comprising:
determining that the weight map indicates a presence of an additional uniform weight on the load cell array;
determining an amount of the additional uniform weight; and
subtracting the uniform weight from each of the plurality of weights.

11. The method of claim 9, wherein identifying the patient location comprises:
capturing an image of the patient on a mattress;
identifying an image location of the patient within the image;
overlaying the image on the weight map; and
identifying a subset of the load cells that overlap with the image location of the patient in the overlaid image.

12. The method of claim 9, wherein identifying a patient location comprises:
receiving a plurality of strain amounts corresponding to a plurality of strain sensors in a strain sensor array;
generating a strain map comprising a plurality of strain amounts associated with a corresponding plurality of X, Y coordinate locations for the plurality of strain sensors;
identifying a patient location based on the strain map; and
identifying a subset of the load cells that correspond to a subset of the strain sensors that overlap with the patient location.

13. The method of claim 12, wherein identifying the patient location comprises identifying a contiguous set of strain values in the strain map that correspond to a predetermined threshold that is indicative of a strain that the patient places on a corresponding plurality of strain sensors.

14. The method of claim 9, wherein determining the weight of the patient comprises summing a subset of the plurality of weights, the plurality of weights being associated with a subset of load cells corresponding to the patient location.
